# EUROPEAN PATENT APPLICATION

(11) **EP 1 832 277 A1**
(43) Date of publication of application: **12.09.2007**
(21) Application number: 07250961.5
(22) Date of filing: 08.03.2007
(51) Int. Cl.: A61K 8/81, A61K 8/92, A61Q 1/10

(54) **Mascara composition with self-emulsifying waxes and latex polymers**

(30) Priority: 10.03.2006 US 373343
(71) Applicant: L'Oréal, 75008 Paris (FR)
(72) Inventor: Feng, Sue, Edison, New Jersey 08820 (US); Kljuic, Ana, New York, New York 10011 (US); Kanji, Mohamed, Edison New Jersey 08837 (US)
(74) Representative: Tanty, François

(57) **Abstract**

Disclosed are mascara compositions containing a latex polymer, a self-emulsifying wax in an amount of at least 15% by weight of the composition, and water, and methods of making and using the compositions.

## Description

### BACKGROUND OF THE INVENTION

The cosmetics industry focuses much of its efforts, in regards to mascara, on increasing two fundamental properties, namely enhancing volume or thickness of eyelashes and extending length of wear. U.S. Patent 5,874,072 teaches mascara containing a mixture of water-insoluble copolymers in the form of an aqueous emulsion with water-soluble film-forming polymers. U.S. Patent 6,248,336 teaches mascara compositions with improved wear characteristics in the form of an emulsion comprising an insoluble polymeric material in an aqueous emulsion, and lipophilic oil components including a polyvinylpyrrolidone/hexadecane copolymer. U.S. Patent 6,534,047 teaches cosmetic compositions for coating keratin fibers, containing a cationic polymer, an anionic polymer and an aqueous dispersion of a C₁-C₆ alkyl (meth)acrylate. The patents teach that the compositions lead rapidly to a uniform make-up result that have good properties of coating, lengthening and curling the eyelashes, as well as good staying power.

U.S. Patent 6,503,521 teaches mascara that enhances volume via the use of three film formers, namely: at least one tacky film former soluble or dispersible in water; at least one tacky film former soluble in oil; and at least one additional water-soluble or water-dispersible film former. U.S. Patent 6,726,917 teaches mascara for providing volume and/or length to eyelashes, containing fibers, pigments, and at least two film formers, including at least one tacky film former soluble or dispersible in water, and at least one tacky film former soluble in oil, chosen from hydrogenated polyisobutenes, adipic acid/diethylene glycol/glycerin crosspolymers, polyethylenes, and polyvinyl laurates.

### SUMMARY OF THE INVENTION

A first aspect of the present invention is directed to a mascara composition that contains a latex polymer, a self-emulsifying wax in an amount of at least 15% by weight of the composition, and water. In some embodiments, the compositions do not contain added emulsifier.

A second aspect of the present invention is directed to a method of preparing a mascara composition that entails preparation of a mixture of a latex polymer, a self-emulsifying wax in an amount of at least 15% by weight of said composition, and water. The mascara composition may be packaged in a suitable container, optionally in combination with an applicator.

A third aspect of the present invention is directed to a method of enhancing length, volume or curl of eyelashes, by applying to the eyelashes a mascara composition containing a latex polymer, a self-emulsifying wax in an amount of at least 15% by weight of the composition, and water.

Due to the presence of self-emulsifying wax, added emulsifiers such as triethanolamine (TEA)-stearate and glyceryl stearate (which are common ingredients in mascara) become optional. Thus, the mascara compositions of the present invention are relatively simple to formulate. The combination of the latex polymer and the self-emulsifying wax is also believed to impart several additional advantages, namely quick length, buildable volume, long wear and curl, creamy texture (with less clumping or thickening over time) and easy removability with water.

### DETAILED DESCRIPTION OF THE INVENTION

One component of the mascara of the present invention is a self-emulsifying wax, which as used herein refers to a chemically modified wax that contains at least one emulsifier component, *e.g.*, a non-ionic emulsifier.

The wax component of the self-emulsifying wax contains one or more cosmetically or dermatological acceptable waxes from among waxes of animal origin, waxes of plant origin, waxes of mineral origin and waxes of synthetic origin. Examples of waxes of animal origin include beeswaxes, lanolin waxes and Chinese insect waxes. Examples of waxes of plant origin include rice waxes, carnauba wax, candellila wax and ouricurry wax, cork fibre waxes, sugar cane waxes, Japan waxes, sumach wax and cotton wax. Examples of waxes of mineral origin include paraffins, microcrystalline waxes, montan waxes and ozokerites. Examples of waxes of synthetic origin include polyolefin waxes, *e.g.*, polyethylene waxes, waxes obtained by Fischer-Tropsch synthesis, waxy copolymers and their esters, and silicone and fluoro waxes. Alternatively, hydrogenated oils of animal or plant origin may be present in the self-emulsifying wax.

Self-emulsifying waxes may be obtained commercially from numerous manufacturers and suppliers. Commercially available self-emulsifying waxes that may be useful in the practice of the present invention include the following: PEG-20 sorbitan beeswax (Atlas G-1726, Uniqema; Nikkol GBW-125, Nikko), PEG-6 beeswax (ESTOL 375, Uniqema), PEG-8 beeswax (Apifil, Gattefosse), PEG-12 beeswax and PEG-12 carnauba wax.

The self-emulsifying wax is present in the mascara in an amount of at least 15% by weight, and ranges generally from 15% to about 50% or more, and in some embodiments from about 20% to about 30%, or about 20% to about 25% by weight of the composition.

Another component of the mascara of the present invention is a latex polymer, which as used herein, refers to an aqueous dispersion of a polymer. Examples of latex polymers useful in the present invention include copolymers of a (meth)acrylic acid and its esters, referred to herein as an (meth)acrylate copolymer. In some embodiments, the copolymer is an acrylates copolymer, which as used herein, refers to a copolymer of two or more monomers selected from the group consisting of acrylic acid, methacrylic acid, and their simple esters, *e.g.*, lower alkyl esters such as methyl, ethyl esters and ethylhexyl esters. Such copolymers, which may be in the form of an aqueous dispersion, are commercially available from numerous sources, including Covacryl A15 and Covacryl E14 by Wackherr, Luviflex Soft from BASF, Luvimer 36D from BASF, Yodosol GH800 from National Starch and Carboset XL-40 from BF Goodrich. In some embodiments, the acrylates copolymer is an aqueous dispersion consisting of the ethyl ester of acrylic acid and the methyl ester of methacrylic acid, and which is commercially available from Daito Kasei under the tradename Daitosol 5000AD (CAS #2135-39-1, which is an aqueous emulsion having a solids content of about 50%).

Other (meth)acrylate copolymers include but are not limited to acrylate copolymers (acrylates/ethylhexyl acrylate copolymer, sold by Daito Kasei under the tradename Daitosol 5000SJ), butyl acrylate/hydroxypropyl dimethicone acrylate copolymers (Granacrysil BAS by Grant Industries, Inc.), acrylates/C12-C22 alkylmethacrylate copolymers (Allianz OPT by ISP), isododecane and acrylates copolymers (Giovarez AC-5099M by Phoenix), and acrylates/octylacrylamide copolymers (Dermacryl-79 by National Starch & Chemical Company).

The amount of the latex polymer present (including the aqueous dispersion in which it may be sold) in the mascara generally ranges from about 0.1 to about 40%, and in some embodiments from about 0.5 to about 30%, or from about 5 to about 20%, by weight of the mascara.

The mascara compositions of the present invention contain some amount of water, generally ranging from about 0.1% to about 50%, and in some embodiments about 1% to about 20%, thus constituting an aqueous phase. In addition to water (and the latex polymer), the aqueous phase may further contain a water-miscible solvent (generally having a miscibility in water of greater than 50% by weight at 25°C), examples of which include lower monoalcohols containing from 1 to 5 carbon atoms such as ethanol or isopropanol, glycols containing from 2 to 8 carbon atoms, such as propylene glycol, ethylene glycol, butylene glycol and dipropylene glycol, C3-C4 ketones and C2-C4 aldehydes.

The inventive compositions may contain any other cosmetically or dermatologically acceptable and, in general, physiologically acceptable oil, such as carbon-based, hydrocarbon-based, fluoro and/or silicone oils, of mineral, animal, plant or synthetic origin, alone or as a mixture, provided that they form a homogeneous and stable mixture along with the other ingredients in the inventive mascara formulations. This ingredient, along with the wax, would constitute a liquid fatty phase of the mascara composition.

Aside from oil, the compositions of the present invention may further contain at least one suitable (*e.g.*, cosmetically or dermatologically acceptable) ingredient, including additives and adjuvants, including, for example, polymers, waxes, thickeners, emulsifiers, moisturizers, colorants, dispersion enhancing agents (e.g., hydrolyzed corn starch), fillers (*e.g.*, powders and Mothers of pearl), fibers, sunscreen agents, preservatives, chelators (such as EDTA and salts thereof, particularly sodium and potassium salts), antioxidants *(e.g.,* BHT, tocopherol), essential oils, fragrances, neutralizing or pH-adjusting agents (*e.g.*, 2-amino-2-methyl-1,3-propanediol (AMPD) and sodium hydroxide), and cosmetically active agents and dermatological active agents, defoaming agents, emollients, vitamins, trace elements and essential fatty acids. These ingredients may be soluble or dispersible in the aqueous or the fatty phase.

Examples of polymers, *e.g.*, film forming polymers, include the following: proteins such as proteins of plant origin, such as wheat or soya bean proteins; gums (*e.g.*, acacia gum); anionic, cationic, amphoteric or nonionic polymers of chitin or chitosan; plant-derived polymers such as hydrolyzed corn starch, cellulose polymers such as hydroxyethyl cellulose, hydroxypropyl cellulose, methyl cellulose, ethyl hydroxyethyl cellulose, carboxymethyl cellulose, and quaternized derivatives of cellulose; anionic polymers including acrylic and methacrylic polymers or copolymers such as polyacrylates or polymethacrylates, and salts (*e.g.*, sodium salts) thereof; vinyl polymers, such as polyvinylpyrrolidones, copolymers of methyl vinyl ether and maleic anhydride, the copolymer of vinyl acetate and crotonic acid, copolymers of vinylpyrrolidone and vinyl acetate; copolymers of vinylpyrrolidone and caprolactam; and polyvinyl alcohols; and quaternized polymers (which are typically cationic polymers, but may also include amphoteric and zwitterionic polymers) such as polyquaternium-4, polyquaternium-6, polyquaternium-7, polyquaternium-8, polyquaternium-9, polyquaternium-10, polyquaternium-22, polyquaternium-32, polyquaternium-39, polyquaternium-44 and polyquaternium-47.

The compositions may contain an additional wax, different from the wax component of the self-emulsifying wax. Cosmetically acceptable waxes suitable for use in the invention are disclosed herein, and include natural and synthetic waxes alike. The wax may be added as a physical blend with one or more emulsifiers *e.g.*, K82H (available from Koster Keunen).

Viscosity may be adjusted by adding an oil phase thickener or an agent useful for gelling a liquid fatty phase. Gelling agents may be chosen from gelling agents in polymeric form and gelling agents in mineral form. The gelling agent may perform its function via chemical reticulation and in some other cases, via physical reticulation. Modified clays may be used as gelling agents, examples of which include hectorites modified with an ammonium chloride of a C₁₀ to C₂₂ fatty acid, such as hectorite modified with distearyldimethylammonium chloride, also known as quaternium-18 bentonite, such as the products sold or made under the names Bentone 34 by the company Rheox, Claytone XL, Claytone 34 and Claytone 40 sold or made by the company Southern Clay, the modified clays known under the name quaternium-18 benzalkonium bentonites and sold or made under the names Claytone HT, Claytone GR and Claytone PS by the company Southern Clay, the clays modified with stearyldimethylbenzoylammonium chloride, known as stearalkonium bentonites, such as the products sold or made under the names Claytone APA and Claytone AF by the company Southern Clay, and Baragel 24 sold or made by the company Rheox. Other mineral gelling agents include silica, such as fumed silica. The fumed silica may have a particle size ranging from about 5 nm to 200 nm.

Water-soluble thickeners or gelling agents that may be used include polyvinylpyrrolidone (PVP), polyvinyl alcohol, crosslinked acrylates (*e.g.* Carbopol 982), hydrophobically-modified acrylates (*e.g.* Carbopol 1382); polyacrylamides such as, for example, the crosslinked copolymers sold under the names Sepigel 305 (CTFA name: polyacrylamide/C13-C14 isoparaffin/Laureth 7) or Simulgel 600 (CTFA name: acrylamide/sodium acryloyldimethyltaurate copolymer/isohexadecane/polysorbate 80) by SEPPIC; 2-acrylamido-2-methylpropanesulphonic acid polymers and copolymers, that are optionally crosslinked and/or neutralized; cellulose derivatives such as hydroxyethylcellulose, sodium carboxymethylcellulose, hydroxypropyl methylcellulose, hydroxypropyl cellulose, ethyl cellulose and hydroxymethyl cellulose; polysaccharides and gums, *e.g.*, natural gums such as xanthan gum, sclerotium, carrageenan and pectin; polysaccharide resins such as starch and its derivatives, hyaluronic acid and its salts, clays, and, in particular, montmorillonites, hectorites, bentonites, and laponites, crosslinked polyacrylic acids, such as the "Carbopol" products from the company Goodrich, the polyglyceryl (meth)acrylate polymers sold under the names "Hispagel" or "Lubragel" by the companies Hispano Quimica or Guardian, crosslinked acrylamide polymers and copolymers, such as those sold under the names "PAS 5161" or "Bozepol C" by the company Hoechst, "Sepigel 305" by the company SEPPIC, crosslinked methacryloyloxyethyltrimethylammonium chloride homopolymers sold under the name "Salcare SC95" by the company Allied Colloid, and associative polymers and, in particular associative polyurethanes.

The thickening/gelling agent is generally present in an amount ranging from about 0.05% to about 20% by weight, and in some embodiments from about 0.5% to about 10% by weight.

To the extent that the composition contains added emulsifier (*i.e.*, other than the emulsifier component of the self-emulsifying wax, as defined herein), it is typically present in an amount below 1.5% by weight of the composition. Emulsifiers suitable for use in the present invention include cosmetically acceptable non-ionic, anionic, cationic and amphoteric emulsifiers.

Compositions of the present invention may also contain a moisturizer. Examples include sodium lactate, mannitol, amino acids, hyaluronic acid, lanolin, urea, petroleum jelly and mixtures thereof. Other examples include polyols such as glycerin, diglycerin, triglycerin, polyglycerin, polyethylene glycol, ethylene glycol, diethylene glycol, triethylene glycol, propylene glycol, dipropylene glycol, hexylene glycol, 1,3-butylene glycol, 1,4-butylene glycol and sorbitol. These agents are present in the compositions of the present invention in amounts generally ranging from about 1.0% to about 15%, and in some cases, from about 2.0% to about 10% by weight of the composition.

Colorants may be chosen from the lipophilic dyes, hydrophilic dyes, traditional pigments, and nacres usually used in cosmetic or dermatological compositions, and mixtures thereof. The coloring agent may have any shape, such as, for example, spheroidal, oval, platelet, irregular, and mixtures thereof. Pigments may optionally be surface-treated e.g., with silicones, perfluorinated compounds, lecithin, and amino acids.

The liposoluble dyes include, for example, Sudan Red, D&C Red 17, D&C Green 6, soybean oil, Sudan Brown, D&C Yellow 11, D&C Violet 2, D&C Orange 5, quinoline yellow and annatto. The water-soluble dyes are, for example, beetroot juice or methylene blue.

The pigments may be chosen from white pigments, colored pigments, inorganic pigments, organic pigments, coated pigments, uncoated pigments, pigments having a micron size and pigments not having a micron size. Among the inorganic pigments that may be mentioned are titanium dioxide, optionally surface-treated, zirconium oxide, zinc oxide, cerium oxide, chromium oxide, manganese violet, ultramarine blue, chromium hydrate, and ferric blue. Among the organic pigments which may be mentioned are carbon black, pigments of D&C type, lakes based on cochineal carmine, lakes based on barium, lakes based on strontium, lakes based on calcium, and lakes based on aluminum.

The nacreous pigments may, for example, be chosen from white nacreous pigments such as mica coated with titanium and mica coated with bismuth oxychloride, colored nacreous pigments such as titanium mica with iron oxides, titanium mica with, for example, ferric blue and/or chromium oxide, titanium mica with an organic pigment of the type mentioned above, as well as nacreous pigments based on bismuth oxychloride, interferential pigments, and goniochromatic pigments.

Colorants are generally present in an amount ranging from about 0.01% to about 50%, and in some embodiments about 0.01% to 30%, about 0.01% to 20%, and about 3% to about 10%, relative to the total weight of the composition.

The compositions of the present invention may also contain dispersion enhancing agents such as polysaccharide resins, *e.g.*, KM 13, available from KAMA International Corp. (Duluth, GA).

Fillers, powders and mothers-of-pearl may also be added to the formulations, typically to modify the texture of the composition and the matteness/gloss effect. Fillers should be understood to mean lamellar or non-lamellar, inorganic or synthetic, colorless or white particles. Mothers-of-pearl should be understood to mean iridescent particles produced especially by certain mollusks in their shell or else synthesized. Representative examples of these ingredients include mica, silica, kaolin, iron oxides, titanium dioxide, polyamide powders, polyamide powders, for instance Nylon® (Orgasol from Atochem), poly-alanine powders, polyethylene powders, tetrafluoroethylene polymer powders, for instance Teflon®, starch (*e.g.*, hydrolyzed corn starch), boron nitride, hollow polymer microspheres such as those of polyvinylidene chloride/acrylonitrile, for instance Expancel® (Nobel Industrie), acrylic powders such as Polytrap® (Dow Corning), polymethyl methacrylate particles and silicone resin microbeads (for example Tospearls® from Toshiba), magnesium hydrocarbonate, hydroxyapatite, hollow silica microspheres (Silica Beads® from Maprecos), and glass and ceramic microcapsules. Filler(s), if present, are in amounts generally ranging from about 0.1% to about 25%, and in some embodiments from about 1% to about 20% by weight of the total weight of the composition.

In some embodiments, the mascara may further contain fibers. The fibers useful in the present invention may be chosen from natural and synthetic fibers. Natural fibers include, but are not limited to, cotton, silk, wool, and other keratin fibers. Synthetic fibers include, but are not limited to, polyester, rayon, nylon and other polyamide fibers. The fibers may be present in the compositions in an amount generally ranging from about 0.01% to about 10% by weight of the composition.

Representative examples of preservatives include alkyl para-hydroxybenzoates, wherein the alkyl radical has from 1, 2, 3, 4, 5 or 6 carbon atoms and preferably from 1 to 4 carbon atoms *e.g.*, methyl para-hydroxybenzoate (methylparaben), ethyl para-hydroxybenzoate (ethylparaben), propyl para-hydroxybenzoate (propylparaben), butyl para-hydroxybenzoate (butylparaben) and isobutyl para-hydroxybenzoate (isobutylparaben), and phenoxyethanol. Mixtures of preservatives are also useful, *e.g.*, the mixture of methylparaben, ethylparaben, propylparaben and butylparaben sold under the name Nipastat by Nipa, the mixture of phenoxyethanol, methylparaben, ethylparaben, propylparaben and butylparaben, also sold by Nipa under the name Phenonip, and the mixture of phenoxyethanol, methylparaben, isopropylparaben, isobutylparaben and butylparaben, sold by ISP under the name Liquapar Optima. The preservative may be present in an amount generally ranging from about 0.01% to about 15% by weight of the composition.

The mascara compositions of the present invention may be applied to eyelashes (including false eyelashes) with a suitable applicator, typically a brush.

The following examples are intended to further illustrate the present invention. They are not intended to limit the invention in any way. Unless otherwise indicated, all parts are by weight.

EXAMPLE 1

| | |
|---|---|
| | |

| **Material** | **Amount by Weight (%)** |
|---|---|
| PEG-8 Beeswax | 22.00 |
| AMPD | 0.50 |
| Carbon Black grind | 22.00 |
| Butylene Glycol | 2.00 |
| Methylparaben | 0.20 |
| Sodium Polymethacrylate | 1.00 |
| Water | 9.19 |
| Polyquaternium-10 | 0.10 |
| | |
| Preservative | 0.01 |
| Daitosol 5000 SJ | 40.00 |
| Alcohol denat. | 3.00 |
| | 100.00 |

This mascara formulation was prepared by the following procedure: melt beeswax at 65-70°C and start homogenizing; add methylparaben and mix for 5 minutes; add carbon black grind slowly and mix for 15-20 minutes; add butylene glycol and mix for 5 minutes; still under heat transfer to paddle mix; add sodium polymethacrylate and mix for 10 minutes; add preservative and mix for 5 minutes; add polyquaternium-10 dissolved in water (prepare this as a side phase) and mix 10 minutes; add AMPD and mix for 5 minutes; at 65°C slowly add Daitosol and mix 5-10 minutes; remove heat and keep mixing; add denatured alcohol at about 45°C; and drop batch at 40-42°C. The carbon black grind (for the mascara exemplified herein) was obtained commercially in the form of a dispersion (Distinctive Cosmetic Ingredients, under the tradename NANOSPERSE INK BLACK LO AQ), which contains carbon black (20%), water (74.4%), acacia (5%), methylparaben (0.3%), and imidazolidinyl urea (0.3%).

EXAMPLE 2

| **Material** | **Amount by Weight (%)** |
|---|---|
| PEG-8 Beeswax | 20.00 |
| Methylparaben | 0.20 |
| Carbon Black grind | 20.00 |
| Butylene Glycol | 3.00 |
| Sodium Polymethacrylate | 1.00 |
| Preservative | 0.70 |
| Water | 2.00 |
| NaOH | 0.10 |
| Water | 8.90 |
| Polyquaternium-10 | 0.10 |
| Daitosol 5000 SJ | 39.00 |
| Alcohol denat. | 5.00 |
| | 100.00 |

This mascara formulation was prepared by the following procedure: melt beeswax at 65-70°C and start homogenizing; add methylparaben and mix for 5 minutes; add carbon black grind slowly and mix for 15-20 minutes; add butylene glycol and mix for 5 minutes; still under heat transfer to paddle mix; add sodium polymethacrylate and mix for 10 minutes; add preservative and mix for 5 minutes; add sodium hydroxide and mix 5 minutes; add polyquaternium-10 dissolved in water (prepare this as a side phase) and mix 10 minutes; at 65°C slowly add Daitosol and mix 5-10 minutes; remove heat and keep mixing; add denatured alcohol at about 45°C; and drop batch to 40-42°C.

Example 3

| Phase | **Material** | **Amount by Weight (%)** |
|---|---|---|
| A1 | PEG-8 Beeswax | 20.00 |
| | C20-40 Alkyl Stearate/emulsifier blend (K82H) | 5.00 |
| | Methylparaben | 0.26 |
| | Black Iron Oxide | 7.00 |
| | Butylene Glycol | 5.00 |
| A2 | Water | 12.88 |
| | Acacia | 1.00 |
| A3 | Sodium Polymethacrylate | 1.00 |
| | Preservatives | 0.76 |
| | NaOH (50% solution) | 0.20 |
| A4 | Water | 10.00 |
| | Polyquaternium-10 | 0.10 |
| A5 | PMMA | 2.00 |
| B | Daitosol 5000 SJ | 29.80 |
| C | Alcohol denat. | 5.00 |
| | | **100.00** |

This mascara formulation was prepared by the following procedure: melt beeswax and C20-40 alkyl stearate at 75-80°C and start homogenizing; add methylparaben and mix for 5 minutes; add black iron oxide and disperse under the homogenizer for 60 minutes; add butylene glycol and mix for 5 minutes; prepare phase A2 by adding acacia to water (which is at room temperature) and mix under heat for 15 minutes to achieve a dispersion; add phase A2 to phase A1 and homogenize for 15 minutes; switch the batch to lightening mixer with paddle still under heat; add sodium polymethacrylate and mix for 10 minutes; add preservatives and mix for 5 minutes; add sodium hydroxide and mix 5 minutes; add polyquaternium-10 dissolved in water (prepare this as a side phase) and mix 10 minutes; (at 65°C) slowly add Daitosol and mix 5-10 minutes; add PMMA (polymethylmethacrylate) and disperse by mixing for 20 minutes; remove heat and keep mixing; add denatured alcohol at about 45°C, and drop batch at 40-42°C.

Example 4

| **MATERIAL** | **AMOUNT BY WEIGHT (%)** |
|---|---|
| PEG 8 Beeswax | 20.00 |
| C20-40 Alkyl Stearate/emulsifier blend (K82H) | 5.00 |
| Methylparaben | 0.20 |
| Carbon Black grind | 20.00 |
| Butylene Glycol | 5.00 |
| Sodium Polymethacrylate | 1.00 |
| Phenonip | 0.70 |
| NaOH (50% solution) | 0.20 |
| Water | 10.80 |
| Polyquaternium-10 | 0.10 |
| PMMA | 2.00 |
| Daitosol 5000 SJ | 30.00 |
| Alcohol denat. | 5.00 |
| | **100.00** |

The mascara composition was prepared in accordance with the following procedure: melt the beeswax and the C20-40 alkyl stearate/emulsifier blend at 75-80°C and start homogenizing; add methylparaben and mix for 5 minutes; add carbon black dispersion slowly and mix for 15-20 minutes; add butylene glycol and mix for 5 minutes; still under heat, transfer the mixture to a paddle mixer; add sodium polymethylmethacrylate and mix for 10 minutes; add Phenonip and mix for 5 minutes; add the sodium hydroxide solution and mix 5 minutes; add polyquaternium-10 dissolved in water (prepare this as a side phase) and mix 10 minutes; at 65°C add PMMA and mix 5 minutes; at 65°C slowly add the Daitosol and mix 5-10 minutes; at 45°C add alcohol; and drop the batch at 40-42°C.

All publications cited in the specification, both patent publications and non-patent publications alike, are indicative of the level of skill of those skilled in the art to which this invention pertains. All these publications are herein incorporated by reference to the same extent as if each individual publication were specifically and individually indicated as being incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A mascara composition comprising a latex polymer, a self-emulsifying wax in an amount of at least 15% by weight of the composition, and water.

2. The mascara composition according to claim 1, wherein said self-emulsifying wax comprises PEG-6 beeswax, PEG-8 beeswax, PEG-12 beeswax, PEG-12 carnauba wax or PEG-20 sorbitan beeswax.

3. The mascara composition according to claim 2, wherein said self-emulsifying wax comprises PEG-20 sorbitan beeswax.

4. The mascara composition according to any preceding claim, wherein said self-emulsifying wax is present in an amount of about 20% to about 30% based on the total weight of said composition.

5. The mascara composition according to claim 4, wherein said self-emulsifying wax is present in an amount of about 20% to about 25% based on the total weight of said composition.

6. The mascara composition according to any preceding claim, wherein said latex polymer comprises an acrylates copolymer.

7. The mascara composition according to claim 6, wherein said latex polymer comprises an ethyl ester of acrylic acid and a methyl ester of methacrylic acid.

8. The mascara composition according to claim 6, wherein said latex polymer comprises acrylates/ethylhexyl acrylate copolymer.

9. The mascara composition according to any preceding claim, wherein said latex polymer is present in an amount of about 0.5% to about 30% based on the total weight of said composition.

10. The mascara composition according to any preceding claim, further comprising a colorant.

11. The mascara composition according to any preceding claim, further comprising a polymer other than said latex polymer.

12. The mascara composition according to any preceding claim, further comprising a neutralizing agent.

13. The mascara composition according to any preceding claim, further comprising added emulsifier.

14. The mascara composition according to any preceding claim, further comprising another wax different from said wax contained in said self-emulsifying wax.

15. The mascara composition according to claim 14, wherein said another wax is C₂₀₋₄₀ alkyl stearate.

16. The mascara composition according to claim 1, wherein said self-emulsifying wax comprises beeswax, said latex polymer comprises an acrylates copolymer or acrylates/ethylhexyl acrylate copolymer, and wherein said mascara further comprises a colorant.

17. A method of preparing a mascara composition, comprising preparation of a mixture of a latex polymer, a self-emulsifying wax in an amount of at least 15% by weight of the composition, and water, under suitable conditions of time and temperature, thus producing the mascara.

18. A method of enhancing length, volume or curl of eyelashes, comprising applying to eyelashes a mascara composition according to any of claims 1 to 16.
